# EUROPEAN PATENT APPLICATION

(11) **EP 3 686 895 A1**
(43) Date of publication of application: **29.07.2020**
(21) Application number: 19153149.0
(22) Date of filing: 22.01.2019
(51) Int. Cl.: G16H 30/00, G16H 50/00

(54) **CONTEXT-DRIVEN DECOMPOSITION FOR NETWORK TRANSMISSION IN MEDICAL IMAGING**

(71) Applicant: Siemens Healthcare GmbH, 91052 Erlangen (DE)
(72) Inventor: RAPAKA, Saikiran, Pennington, NJ New Jersey 08534 (US)
(74) Representative: EIP

(57) **Abstract**

Context-driven decomposition is provided for network transmission (14) in medical imaging. The context of the imaging, such as detection (11) of specific pathology, is used to prioritize data transfer (14). The data for a region of interest is sent before and separately from data for other regions. By detecting (11) an abnormality or using other context, the data for image processing related to that abnormality is sent (14) before sending other data from a same scan. The server (38) receives the data of interest more rapidly and may even return (16) results before the transfer of other data from the scan is completed.

## Description

### BACKGROUND

The present embodiments relate to cloud-assisted image processing. Many medical image analysis algorithms are migrating to cloud environments due to multiple advantages, including the ability to rapidly provision and scale computation capabilities, faster and more controllable deployment of advanced models, and the ability to learn from larger collections of data. As these applications move to the cloud, basic limitations such as the network bandwidth start to become more critical in delivering fast results. For many applications, it can take a few minutes just to have the data reach the cloud server before the model computes results.

Current applications either do not address this problem directly or use different "off-the-shelf" compression techniques, such as zip or JPEG. Even with such compression, the magnitude of speed up in data transfer is relatively limited.

### SUMMARY

By way of introduction, the preferred embodiments described below include methods, systems, instructions, and non-transitory computer readable media for context-driven decomposition for network transmission in medical imaging. The context of the imaging, such as detection of specific pathology, is used to prioritize data transfer. The data for a region of interest is sent before and separately from data for other regions. By detecting an abnormality or using other context, the data for image processing related to that abnormality is sent before sending other data from a same scan. The server receives the data of interest more rapidly and may even return results before the transfer of other data from the scan is completed.

In a first aspect, a method is provided for context-driven decomposition for network transmission in medical imaging. The anatomical context is used to decompose the data into parts for transmission. A medical imaging system scans a patient, providing scan data for a volume of the patient. An image processor of the medical imaging system segments an anatomic structure of a patient represented in scan data. The scan data of the anatomic structure includes first part of the scan data based on the segmenting. The scan data is transmitted in at least the first part and a second part to one or more remote servers. The one or more remote servers are remote from the medical imaging system. The transmitting prioritizes the transmitting of the first part before the second part. A first result is received from the remote server based on image processing of the first part and is displayed.

The method of this aspect may include detecting an anatomy of interest from the scan data. The segmenting includes segmenting the anatomic structure based on the detecting of the anatomy.

The method of this aspect may include separately compressing the scan data of the first and second parts. The transmitting includes transmitting the first and second parts as compressed.

The method of this aspect may include receiving a second result from the remote server based on image processing of the second part.

In one embodiment, the receiving the first result occurs prior to completion of the transmitting of the second part.

In another embodiment, the segmenting includes segmenting with a machine-learned network.

As another embodiment, segmenting the anatomical structure includes segmenting a first organ or a first portion of the first organ. The first part is the scan data representing the first organ or the first portion of the first organ. The second part is the scan data representing a segmented second organ or a second part of the first organ.

In yet another embodiment, transmitting includes transmitting the first part with information in addition to the scan data. Receiving includes receiving the first result based on the image processing of the first part and based on the information.

As yet another embodiment, transmitting includes transmitting the first part to a first remote server and the second part to a second remote servers. The first and second remote servers are selected based on the anatomic structure in the first part and based on another anatomic structure in the second part.

The method of this aspect may include receiving a request from the one or more remote servers for additional information and providing by the medical imaging system the additional information.

In a second aspect, a method is provided for context-driven decomposition for network transmission in medical imaging. An image processor detects an abnormality represented in image data of a patient and segments a first region of interest in the image data based on the detecting of the abnormality. The image data for the region of interest is transmitted as a file separate from the image data for outside the region of interest to a server, which is remote from the image processor. A quantity, classification, segmentation, ranking, or combinations thereof is received from the server for the abnormality based on the image data for the region of interest and displayed.

In one embodiment of this method, detecting includes detecting the abnormality in an organ by a first machine-learned network. Segmenting includes segmenting the organ by a second machine-learned network.

In another embodiment, segmenting includes segmenting the region of interest and other regions of interest. Transmitting includes transmitting the image data for the region of interest as the separate file and transmitting the image data for the other regions of interest in other files sequentially. The separate file for the region of interest is transmitted before the other files.

As another embodiment, transmitting includes transmitting the file with information in addition to the image data. Receiving includes receiving the quantity, classification, segmentation, ranking, or combinations thereof based on image processing of the image data of the region of interest and based on the information.

In yet another embodiment, transmitting includes transmitting to the server comprises selecting the server from a group of servers based on the region of interest.

The method of this aspect may include receiving a request from the server for additional information and providing by the image processor the additional information.

In a third aspect, a system is provided for context-driven decomposition for network transmission in medical imaging. A medical imaging system is configured to scan a patient, divide the scan data into blocks based on anatomy of the patient, and transmit the blocks in an order from anatomical context of the scan data for the blocks. The medical imaging system is at a medical facility. A server connects to the medical imaging system through a network and outside the facility. The server is configured to receive the blocks of scan data, image process each block separately, and return information from the image process for each block to the medical imaging system.

In one embodiment, the medical imaging system is configured to transmit the blocks as separate files. The anatomical context is a detection of an abnormality in anatomy so that the block representing the abnormality is transmitted before blocks for other organs.

In another embodiment, the medical imaging system is configured to divide by segmenting based on the anatomy and configured to transmit the block with the order based on detection of an abnormality in anatomy.

In yet another embodiment, the server is configured to request additional scan data relative to one of the blocks. The medical imaging system is configured to provide the additional scan data in response to the request.

The present invention is defined by the following claims, and nothing in this section should be taken as a limitation on those claims. Further aspects and advantages of the invention are discussed below in conjunction with the preferred embodiments and may be later claimed independently or in combination. Any method acts may be performed by a system, and any system acts may be performed in a method. Non-transitory computer readable media may be provided with instructions for performing any of the acts of the method or implementing configuration of the system using a processor.

### BRIEF DESCRIPTION OF THE DRAWINGS

The components and the figures are not necessarily to scale, emphasis instead being placed upon illustrating the principles of the invention. Moreover, in the figures, like reference numerals designate corresponding parts throughout the different views.
Figure 1 is a flow chart diagram of one embodiment of a method for context-driven decomposition for network transmission in medical imaging;
Figure 2 illustrates example division of scan data and prioritized transmission to the cloud based on context of the scan data; and
Figure 3 is one embodiment of a system for context-driven decomposition for network transmission in medical imaging.

### DETAILED DESCRIPTION OF THE DRAWINGS AND PRESENTLY PREFERRED EMBODIMENTS

Context-driven acceleration is provided for networked image processing applications. For data compression, the context of use and past knowledge is accounted for in delivering rapid throughput. The delivery of clinically useful results is accelerated using context or a model-based approach for context-based analysis. For example, artificial intelligence identifies landmarks and regions of interest rapidly. For compression, only the relevant information is uploaded to the server for processing. In many cases, the total data to be uploaded can be reduced by a factor of 100, without affecting the quality of the results. For acceleration of results, the data for regions of interest are uploaded first so that results may be returned without waiting for upload of all the data.

The knowledge of anatomical content in the medical image is exploited as context to speed up data transfer. For instance, if the application only needs the coronary information from an image, by transmitting the content of the coronary regions before the rest of the image, delivery of results is accelerated. Similarly, medical images may be decomposed into different regions corresponding to different organs, and the information may be communicated to the server in an order which depends on the importance of each region for a specific application. In typical applications, the user may be presented with results of analysis significantly faster. In more complex applications, the user may be presented with results for one of the organs, while the data for another organ is being uploaded instead of waiting for the entire image to upload in order to provide all results simultaneously. Significantly faster turn-around times, at least for some results, are provided for cloud applications.

As a concrete example of possible acceleration, a routine Calcium Scoring CT scan is performed. A typical image has of over 26 million voxels (e.g., image size of about 512x512x100) with an average voxel size of about 0.16 mm³, corresponding to a total scanned volume of about 4.0 L. The coronary arteries of are particular interest for calcium scoring. Considering that the total volume of coronary arteries is estimated to be about 4.0 mL, the ratio of the total volume in the image to that in the coronaries is more than a factor of 1000. Even assuming oversampling in the coronary region by a factor of 10, the data transfer is accelerated by a factor of about 100 for calcium scoring. In this example, the speed up in providing a calcium scoring result from a cloud server to the user is achieved by focusing transmission of data to the server on the context of interest - analyzing the coronary arteries. A considerable volume of the image contains other organs, such as the lungs and the ribs. In one setup, the other organs are ignored by the models running on the server side (i.e., this data is not uploaded to the server for analysis). In a different setup, the data for other organs is uploaded either during or after the analysis of the coronary arteries for image processing by the server.

Such an approach can also be useful for other cardiovascular topics, such as plaque quantification and estimation of functional significance of coronary artery disease. Non-cardiovascular topics may benefit, such as where imaging is provided for any part of the body (e.g., torso, liver, arm, leg, head, or neck). Other organs or anatomic structures may be used. Other modalities of imaging with more or less data, volume, and/or relative differences in size of interest may be used.

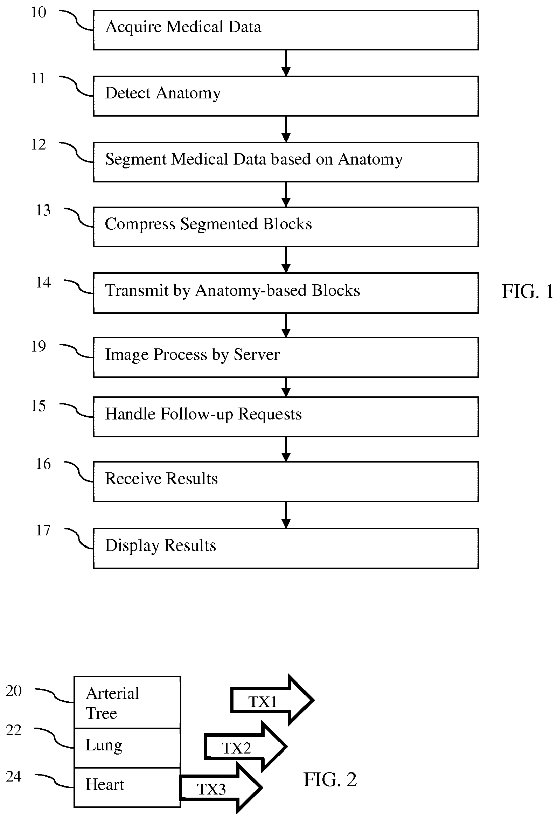

Figure 1 is a flow chart diagram of one embodiment of a method for context-driven decomposition for network transmission in medical imaging. The context of the scanning is used to determine which data and/or the order of data to be sent to a cloud server for image processing. The context may be provided by detection of an abnormality, the type of medical scan being performed, the anatomy represented in the scan data, and/or other indication of an anatomical region of interest. This context is used to accelerate results of interest from a remote server by providing the appropriate data before other data.

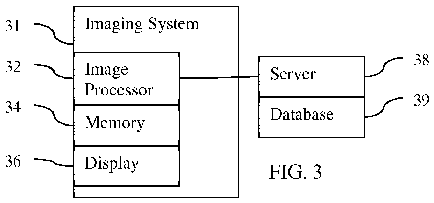

The method is implemented by the system of Figure 3 or another system. For example, the method is implemented on a computer or processor associated with a magnetic resonance (MR), computed tomography (CT), ultrasound, emission, x-ray or other imaging system. As another example, the method is implemented on a picture archiving and communications system (PACS) workstation or server. The acquisition of the medical data is performed by an imaging system or PACS system. The imaging system, PACS system, or other imaging workstation identifies data to be transmitted to a remote or cloud server based on context. The remote or cloud server performs image processing and provides results back to the medical imaging system (e.g., imaging system or scanner or workstation) for output to the user.

The acts are performed in the order shown (e.g., top to bottom) or other orders. Additional, different, or fewer acts may be provided. For example, the method is performed without display in act 17, reception in act 16, follow-up handling in act 15, compression in act 13, and/or detection in act 11. As another example, acts for receiving user input, providing additional information from a memory, database, or sensors, and/or combinations thereof are provided. In yet another example, act 19 is not provided (e.g., is performed by a different device).

In act 10, a medical imaging system acquires scan data. The medical imaging system is a workstation, computer, and/or medical scanner. For example, a workstation or computer acquires scan data from memory or from a medical scanner. As another example, the medical scanner acquires scan data by scanning the patient. Any type of medical scanner may be used, such as CT, MR, ultrasound, PET, SPECT, or x-ray.

The scan data is a frame of data representing the patient. The data may be in any format. While the terms image and imaging are used, the image or imaging data may be in a format prior to actual display of the image. For example, the medical image may be a plurality of scalar values representing different locations in a Cartesian or polar coordinate format different than a display format. As another example, the medical image may be a plurality red, green, blue (e.g., RGB) values output to a display for generating the image in the display format. The medical image may be currently or previously displayed image in the display or other format. The image or imaging is a dataset that may be used for imaging, such as scan data representing the patient. Similarly, scan data may include data from a scan of the patient with any amount of image processing from scanning to generating an image on a display.

Any type of scan data may be used. In one embodiment, the scan data is a chest CT image acquired with a CT system. For example, a chest CT dataset may be used for detecting a bronchial tree, fissures, and/or vessels in the lung. As another example, MR data representing a patient is acquired. MR data is acquired with an MR system. The data is acquired using an imaging sequence for scanning a patient. Data representing an interior region of a patient is acquired. For MR, the measurement data is k-space data. Fourier analysis is performed to reconstruct the data from the k-space into a three-dimensional object or image space. For CT, the raw data is reconstructed into a three-dimensional representation.

The scan data represents a one, two, or three-dimensional region of the patient. For example, the scan data represents a volume of the patient. Values are provided for each of multiple locations distributed in two or three dimensions. The medical image is acquired as a frame of data. The frame of data represents the scan region at a given time or period. The dataset may represent the area or volume over time, such as providing a 4D representation of the patient.

In act 11, the image processor detects an anatomy of interest from the scan data. The anatomy of interest is anatomical structure, organ, part of a patient (e.g., landmark), lesion, artery calcification, lung nodules, deformity, or another pathology. In alternative embodiments, a foreign object (e.g., medical instrument) is detected. An abnormality (e.g., foreign object or pathology) may be detected.

The detection identifies the existence of the anatomy of interest. The scan data is processed to detect that an anatomy of interest is within the patient. The type of scan or reason for the scan may indicate an expected anatomy of interest. The image processing confirms the existence of the expected anatomy and/or severity. Alternatively, the image processor searches for one or more different anatomies of interest in order to determine whether the anatomies of interest exist in the patient.

Any now known or later developed approach to detect the anatomy of interest or other abnormality may be used. For example, image processing is performed on the scan data to detect. Filtering, such as directional filtering, may be used. Template matching, thresholding, and/or other image processing is applied to the scan data using an algorithm to detect. In one embodiment, a machine-learned network is applied. The scan data or values of features derived from the scan data (e.g., Haar wavelets) are input to a machine-learned network, such as a neural network, support vector machine, and/or Bayesian network. The machine-learned network outputs a classification or detection results, such as positive or negative for a given abnormality or anatomy of interest.

The ordering of which data is to be sent to the server starts with detection. The relatively fast machine-learned models are performed locally or on the client side (e.g., at a PACS workstation or medical scanner) in a manner of seconds. Applying an algorithm may take minutes or hours. For instance, a machine-learned model is applied to the image of a patient in the client side to detect the presence of lung nodules and/or calcification in the coronary arteries. In this case, the upload of the lung and coronary data to a remote server may be prioritized to provide quantitative evaluation with a more detailed model, followed by upload of the image data for other organs.

The image processing may detection one or more anatomies of interest or abnormalities. For example, one machine-learned network may detect multiple different types of abnormalities. Alternatively, different image processing is applied to detect different abnormalities and/or anatomies of interest. Different image processing or detectors may be used for different organs.

The detection triggers the segmentation of act 12. In one embodiment, the detection is performed as part of segmentation. The detection includes the location of the anatomy of interest and/or abnormality. Alternatively, an organ is segmented, and then detection is applied to the segmented scan data. In yet other embodiments, detection is not performed. The scan data is segmented, and context for ordering transmission is derived from another source (e.g., size of segmented organ, type of medical scan, and/or medical record of the patient).

The image processor of the medical imaging system segments the anatomic structure of the patient represented in the scan data. The scan data is divided into different parts, such as the segmented part (e.g., volume of interest) and the remaining part (e.g., volume outside the volume of interest) or multiple segmented parts (e.g., different organs). The segmentation creates the two parts (e.g., division) and/or separate parts are separately segmented (e.g., three or more parts). The different parts are formed from scan data representing different locations. Different sets of data are formed.

The segmentation is based on the detection of the anatomy of interest and/or abnormality. For example, calcification is detected. The segmentation is then of the arteries, such as the arterial tree or part of the arteries associated with or in which the calcification occurs. Based on the detection, the detected anatomy of interest, detected abnormality, organ in which the detected object occurs, and/or volume larger than and enclosing the detected object is segmented. In alternative embodiments, the segmentation is performed regardless of detection. For example, one or more organs are segmented based on the type of scan (e.g., chest CT).

Any now known or later developed segmentation may be used. Thresholds may be applied. A model or template may be fit. Random walker algorithm or gradient-based segmentation may be used. In one embodiment, a region growing and skeletonization approach is used. One or more seeds are located by the processor or manual entry. The seed or seeds are used in region growing to find locations of the object. Skeletonization may be used to model a tree structure, such as using lines to represent the centers of the branches of the bronchial tree or vessels. Since the segmentation is for transmission by part, the accuracy of the segmentation may be less and/or err by providing a volume around the object of interest rather than exact boundaries. The segmentation covers the region of interest. The segmentation may be a coarse localization of the organ or an over-segmentation. In other words, the model on the client side does not have to be super accurate.

In one embodiment, a machine-learned network is used to segment. The image processor applies a trained neural network, support vector machine, or other machine-learned network. The scan data or values for features derived from the scan data are input to the machine-learned network. The machine-learned network outputs the segmentation, such as a sphere or prism surrounding an organ.

The locations of the anatomy of interest, abnormality, and/or surrounding volume (e.g., organ) represented by the data are found, such as identifying the outer boundary and/or volume. The locations provide the segmentation encapsulating the anatomic structure or abnormality. Alternatively, the data and/or locations are separated or isolated form other data as the segmentation.

The segmentation decomposes the image data into one or more regions of interest based on anatomical and/or clinical features. For instance, a chest CT scan may be decomposed into regions corresponding to the lungs, airways, vertebrae, aorta, cardiac muscle, coronary arteries, etc. As another example, the chest CT scan is decomposed into coronary arteries and non-coronary arteries (e.g., all other locations). The scan data may be segmented into different organs, parts of organs, groups of organs, and/or remaining (e.g., non-segmented) locations. The parts may be a segmented anatomical region for one part and some surrounding region for the other part. The parts may be a segmented region at one resolution for one part and data forming a greater resolution for that region for the other part. The volume or area represented by the scan data is divided into sub-volumes or sub-areas.

Two or three spatial dimensions define the segmented parts. Parts with additional information, such as one or more measures (e.g., distance from gross structure, velocity of any motion in the part, and/or elasticity) for each part may be provided, resulting in additional dimensions of the data. The parts may be of any dimension. The parts may be medical image data or other data, such as an extracted surface provided as mesh data representing one or more surfaces. Filtering may be provided, such as low pass filtering before dividing into parts or separate filtering applied to the parts.

The parts are spatially distinct with or without overlap. For example, each voxel is included in only one part. Different groups of voxels are provided in different parts. Alternatively, one or more voxels may be included in multiple parts.

In act 13, the image processor compresses the scan data and/or other data of each part. The segmented parts are compressed independently. Alternatively, the segmented parts as divided are not compressed. The parts are provided as different collections and/or files.

Any compression may be used, such as JPEG, zip, or 7z. Each part is formatted into a different file or collection of data. Each part may be separately compressed. The same compression is used but is separately or independently applied to the data of each part. As a result, the compressed data may be uncompressed without data from other parts.

In act 14, the image processor, using a network interface, transmits the scan data for one part separately from other parts. The scan data is formatted into different files or collections. The transmission of each part is transmission of the file (e.g., separately compressed file) or part. Each part may be transmitted in one or more packets using TCP/IP computer network communications. The packets for a same file or part share a link or header information for reassembling. The packets for different files or parts do not share the link or header for reassembling. Some header or metadata may be shared for the different parts so that the parts are linked but are formatted for separate transmission. The transmission for a given region of interest is of a file separate from image data for outside the region of interest or for other parts.

The transmission is from a local workstation or medical scanner to one or more remote servers. A client-service configuration is used. The client is the local image processor, and the service is provided by a remote server. The server is remote by being in a different facility, city, county, state, and/or country. Alternatively, the server is remote by being in a separate room so that computer network communications are needed between the image processor and the server.

The image processor prioritizes the transmissions. One part is transmitted before another part. For example, the file with compressed scan data for the arteries is transmitted before the file with compressed scan data for the lungs. The different files are transmitted sequentially. The image data for the different regions of the patient are sent one after the other with no or some delay in between. There may be some overlap (e.g., sending packets for the later part before sending all packets for the earlier part) but the transmission starts sending the earlier part first. The sending of results for the second part does not have to be after receiving the results for the first part. The sending and receiving are independently done (for instance, using multi-threading) and may overlap with each other. The transmission of parts does not have to be synchronized There may be delay for acknowledgement of completion of receipt and/or completion of server processing before beginning transmission of the next file. In an alternative embodiment, the priority is to send one part and not another part.

The scan data is uploaded for each region of interest sequentially, so that the user may already be interpreting the results for the earlier transmitted region while a different region is being uploaded. Similarly, another example is analysis of oncological samples - for instance, a liver lesion. The location of a lesion is localized by segmentation, and the image data corresponding to the lesion is uploaded first, before uploading the scan data from the rest of the medical image. Figure 2 shows an example where the scan data is segmented into an arterial tree part 20, a lung part 22, and a heart part 24. The arterial tree part 20 is associated with detected calcification or stenosis, so is transmitted first (TX1). The lung may include nodules, so the lung part 22 is transmitted next (TX2). No abnormalities are detected in the heart, so the heart part 24 of the scan data is transmitted last (TX3) .

The data being transmitted is scan data. Information in addition to the image data may be transmitted in the file or linked to the file. For example, information about the patient and/or scan settings are transmitted. Patient information may include demographics, genetic, blood biomarker, test results, sensor readings, heart rate, blood pressure, and/or other clinical information. Any metadata may be provided, such as information about the organ and/or abnormality of interest. In one embodiment, a prediction or characteristic based on local processing is included. For example, the segmentation, detection, and/or application of other image processing may provide a measure, estimate, prognosis, and/or diagnosis. The results from this image processing is provided as additional patient information with or in addition to the scan data.

In one embodiment, the uploaded data contains either predictions from other models (e.g., those running on the client side) or any other information that might be useful for the models running at the server. In one example, when the coronary region of the chest CT image is uploaded, the metadata specifies that coronary calcium was detected, where and how much of the calcium was estimated, that lung nodules are most likely present, any past medical problems, current regiment of the patient, biomarkers which might be relevant, etc.

The parts are transmitted as compressed. The compressed files are transmitted. In alternative embodiments, the data is sent without compression.

The image processor sends the different parts to the same server. Alternatively, different parts are sent to different servers. For a given part, a server from a group of servers is selected to receive the part. The selection is based on the part, demand, availability, and/or other information. For example, different servers provide different services, such as applying models or image processing for different organs. The organ or region of interest associated with the part is used to select the server. Data for different parts of the scanned volume are sent to different servers. The anatomic structure represented in the part is used to select the remote server to which that part is transmitted.

By decoupling the upload process into organ-based regions of interest, different machines (e.g., servers) may host the models for each organ. The target machine or server where the data is being uploaded need not be the same as another server to which other data is sent. This enables rapid scaling of the computational infrastructure of the servers based on demand. Pathology with greater demand for server-based image processing may be assigned a server with greater processing capability and/or a larger bank of servers. In this way, the computation dedicated to each organ may be proportional to the actual incidence rate of pathology in that organ.

In act 19, the remote server provides imaging processing. The remote server may provide the same type of image processing as performed by the image processor. For example, detection and/or segmentation is performed but with different settings, a different machine-learned network, and/or a different process. A more computationally demanding or thorough image process may be provided. Alternatively, the remote server provides additional or different image processing, such as providing a therapy plan, prognosis, diagnosis, identification of similar case, or other prediction different than provided by the image processor.

The remote server receives the transmitted data. Any compression is reversed. The transmitted data itself may include a request for services or a separate request is received. The image processor then performs further image processing on the data and transmits a result. The server provides cloud-based support for the classification or other image processing in a service-based model. A single cloud-based platform may be improved and maintained for the benefit of several services and applications. For example, a provider service trains, retrains, and/or uses a machine-learned classifier to serve measurements, therapy recommendation, related case information, prognosis, and/or diagnosis to customers operating local machines. For example, a hospital or radiologist group contracts for classification. Using a local imaging system, the parts are extracted and sent to the service. The service returns classifications for the different parts where the classifications are to be used locally.

For completing the image processing by the server, the server may request additional information in act 15. For example, the local segmentation may not include enough data (e.g., volume restricted to only a part of a vessel tree or not including adjacent tissue to an organ of interest). The server performs segmentation and identifies that missing information may help further image processing based on the results of the segmentation. The server then requests additional data from the local image processor.

The image processor of the local medical imaging system (e.g., of the medical scanner or PACS workstation) receives the request from the remote server or requests from servers. The requests are for additional information. In response, the image processor segments out or identifies the additional information. The additional information is then transmitted to the server or servers by the image processor.

Once the information for the organs of interest is uploaded, the model running on the server side may request further information to do an accurate computation on a case-specific basis. For example, the client-side model may not send the entire organ of interest, so the server requests that additional data covering a larger space be sent. For instance, if the patient is suffering from an abnormal sized organ, the client-side model may not be able to accurately isolate or segment the organ of interest. The server-side model, being more detailed and computationally powerful, may recognize this issue and request more data. As another example, the influence of other pathologies may interact with the disease, so data for the other pathology is requested. For instance, the metadata (e.g., biomarkers) sent with the image data may highlight the potential presence of other diseases, which might influence the prediction for the current organ of interest. The scan data that may reflect information about the other diseases may be requested.

In act 16, the image processor of the local imaging system receives a result or results from the remote server. The results from the image processing of the server are provided back to the local image processor by the server. The result or results based on image processing of just the scan data of one part or segment are received. The result or results based on image processing the scan data and additional information (e.g., metadata) for one part or segment are received.

Any results may be received. For example, a quantity, classification, segmentation, ranking, prognosis, diagnosis, identification of similar patient, or combinations thereof are received from the server for a detected abnormality. The result may be that the organ is normal. The received results are based on the image data for the region of interest about the abnormality.

The results for one part may be received before completion of transmitting another part. For example, results regarding calcification in arteries are received from the server before sending scan data for the lungs or other organ. The segmented scan data for the arteries and/or surrounding tissue are used to provide results prior to initiating or completing sending of a next or last part of the scan volume.

The results from server-based image processing of scan data for other parts are received. The results for the different parts are received at different times due to transmission and imaging processing in sequence. Alternatively, the server collects the results for the different parts and provides the results back in a group or as one file. In another embodiment, differences in time to image process may result in receipt of results in a different order than transmitted parts.

In act 17, the image processor, using a display, displays the result or results provided by the server or servers. For example, the quantity, classification, segmentation, ranking, prognosis, diagnosis, similar patient, or combinations thereof are displayed. An image of the patient (i.e., of anatomy), with or without segmentation, is displayed with annotation, highlighting, or other indication of the results of the image processing. The local image processor may use the results to derive further information, which is displayed to the user. A patient medical record including the results may be displayed.

The image is displayed on a display of a medical imaging system, such as an MR or CT system. Alternatively, the image is displayed on a workstation, computer or other device. The image may be stored in and recalled from a PACS memory.

Figure 3 shows a system for context-driven decomposition for network transmission in medical imaging. The system includes an imaging system 31 with a memory 34, an image processor 32, and a display 36, and includes a server 38 with a database 39. Additional, different, or fewer components may be provided. For example, a network or network connection is provided, such as a TCP/IP computer network for networking with a medical imaging network or data archival system. In another example, a user interface is provided. As another example, the server 38 and database 39 are not provided. In other examples, the server 38 connects through a network with many imaging systems 80 and/or processors 82. In yet other examples, multiple servers 38 communicatively connect with the imaging system 31.

The image processor 32, memory 34, and display 36 are part of the imaging system 31. Alternatively, the image processor 32, memory 34, and display 36 are part of an archival and/or image processing system, such as associated with a medical records database workstation or server, separate from the imaging system 31. In other embodiments, the image processor 32, memory 34, and display 36 are a personal computer, such as desktop or laptop, a workstation, or combinations thereof. The image processor 32, display 36, and memory 34 may be provided without other components for acquiring data by scanning a patient.

The imaging system 31 is a medical diagnostic imaging system. Ultrasound, computed tomography (CT), x-ray, fluoroscopy, positron emission tomography (PET), single photon emission computed tomography (SPECT), and/or magnetic resonance (MR) systems may be used. The imaging system 31 may include a transmitter and includes a detector for scanning or receiving data representative of the interior of the patient.

In one embodiment, the imaging system 31 is a CT system. An x-ray source is connected with a gantry. A detector is also connected with a gantry opposite the x-ray source. The patient is positioned between the source and detector. The source and detector are on opposite sides of the patient and rotate and/or translate about the patient. The detected x-ray energy passing through the patient is converted, reconstructed or transformed into data representing different spatial locations within the patient.

In another embodiment, the imaging system 31 is a MR system. The MR system includes a main field magnet, such as a cryomagnet, and gradient coils. A whole body coil is provided for transmitting and/or receiving. Local coils may be used, such as for receiving electromagnetic energy emitted by atoms in response to pulses. Other processing components may be provided, such as for planning and generating transmit pulses for the coils based on the sequence and for receiving and processing the received k-space data. The received k-space data is converted into object or image space data with Fourier processing.

The imaging system 31 is located at a medical facility. For example, the imaging system 31 is in a room dedicated for scanning or imaging patients. A base or other part of the imaging system 31 is fixed to a floor or ceiling of the room. A portable imaging system 31 may be provided, such as allowing the imaging system 31 to be moved to different rooms in the medical facility. The medical facility may be a hospital, imaging center, physician office, or other building for treatment or diagnosis of patients.

The memory 34 may be a graphics processing memory, a video random access memory, a random-access memory, system memory, cache memory, hard drive, optical media, magnetic media, flash drive, buffer, database, combinations thereof, or other now known or later developed memory device for storing data or video information. The memory 34 is part of the imaging system 31, part of a computer associated with the image processor 32, part of a database, part of another system, a picture archival memory, or a standalone device.

The memory 34 stores medical imaging data representing the patient, segmentation information, locations of anatomy and/or anatomical structure, detection information, classification results, a machine-learned network, and/or images. The memory 34 may alternatively or additionally store data during processing, such as storing compressed files, detected boundaries, images, graphic overlays, quantities, or other information discussed herein.

The memory 34 or other memory is alternatively or additionally a non-transitory computer readable storage medium storing data representing instructions executable by the programmed image processor 32 for use in medical imaging. The instructions for implementing the processes, methods and/or techniques discussed herein are provided on non-transitory computer-readable storage media or memories, such as a cache, buffer, RAM, removable media, hard drive or other computer readable storage media. Non-transitory computer readable storage media include various types of volatile and nonvolatile storage media. The functions, acts or tasks illustrated in the figures or described herein are executed in response to one or more sets of instructions stored in or on computer readable storage media. The functions, acts or tasks are independent of the particular type of instructions set, storage media, processor or processing strategy and may be performed by software, hardware, integrated circuits, firmware, micro code and the like, operating alone, or in combination. Likewise, processing strategies may include multiprocessing, multitasking, parallel processing, and the like.

In one embodiment, the instructions are stored on a removable media device for reading by local or remote systems. In other embodiments, the instructions are stored in a remote location for transfer through a computer network or over telephone lines. In yet other embodiments, the instructions are stored within a given computer, CPU, GPU, or system.

The image processor 32 is a general processor, central processing unit, control processor, graphics processor, digital signal processor, three-dimensional rendering processor, application specific integrated circuit, field programmable gate array, artificial intelligence processor, digital circuit, analog circuit, combinations thereof, or other now known or later developed device for detection, segmentation, transmission, receipt of results, display of results, or another act. The image processor 32 is a single device or multiple devices operating in serial, parallel, or separately. The image processor 32 may be a main processor of a computer, such as a laptop or desktop computer, or may be a processor for handling some tasks in a larger system, such as in an imaging system. The image processor 32 is configured by instructions, design, hardware, and/or software to perform the acts discussed herein.

The image processor 32 is configured to divide the scan data into blocks based on anatomy of the patient. The anatomy or scan volume is segmented into different parts. Detection and/or segmentation are applied, resulting in identifying different portions of the entire scan volume. The scan volume is separated into blocks based on the identified objects in the scan volume.

The image processor 32 is configured to transmit the blocks. Each block is formed as a separate file or data structure for separate or independent transmission. The blocks may be separately compressed for transmission.

The blocks are scheduled for and transmitted in an order. The order is based on the anatomical context of the scan data for the blocks. The anatomical context is the anatomy of interest and/or associated with an abnormality. The type of scan, initial diagnosis, test results, symptoms, or pathology may provide context for what region of the patient is of particular interest. The block corresponding with that region is segmented and scheduled for transmission before other blocks. For example, a block representing a detected abnormality is transmitted first or before another block for another organ or region. The order of transmission of the blocks is based on detection of one or more abnormalities in anatomy or another context.

The image processor 32 may be configured to provide the additional scan data in response to a request. The image processor 32 may interact with the server 38 for any follow-up requests for additional scan data, such as for surrounding locations due to co-morbidities in other organs or insufficient local segmentation. The image processor 32 may be configured to initially provide metadata or non-scan data to be used by the server with the scan data of one or more blocks. The metadata may be added to the block for compression with the scan data or may be transmitted separately.

The display 36 is a monitor, LCD, projector, plasma display, CRT, printer, or other now known or later developed devise for outputting visual information. The display 36 receives images, graphics, text, quantities, or other information from the image processor 32, memory 34, imaging system 31, or the server 38. One or more medical images are displayed. The images are of a region of the patient. The image includes an indication, such as a graphic or colorization, of the located anatomical structure, detected abnormality, diagnosis, prognosis, classification, or other result from the server and/or local imaging processing. Alternatively or additionally, the image includes a quantity based on measurement from the scan data. The results may be displayed as the image without the medical image representation of the patient.

The server 38 is a processor or group of processors. More than one server 38 may be provided. The server 38 connects to the imaging system 30 through a network. The server 38 is outside of a facility housing the imaging system 30, such as in a server facility or at a provider facility different than the medical facility hosting the imaging system 30.

The server 38 is configured by hardware, firmware, and/or software to receive blocks of scan data, decompress each block as received, image process each block separately, request additional scan data relative to one or more of the blocks, and/or return information from the image processing for one or more of the blocks (e.g., for each of the blocks) to the medical imaging system 30. To image process, the server 38 either applies a machine-learned network or uses a programmed algorithm.

The database 39 is a memory, such as a bank of memories, for storing machine-learned networks, blocks of data, requests, additional information, and/or image processing results.

While the invention has been described above by reference to various embodiments, it should be understood that many changes and modifications can be made without departing from the scope of the invention. It is therefore intended that the foregoing detailed description be regarded as illustrative rather than limiting, and that it be understood that it is the following claims, including all equivalents, that are intended to define the spirit and scope of this invention.

## Claims

1. A method for context-driven decomposition for network transmission in medical imaging, the method comprising:
scanning (10), by a medical imaging system (31), a patient, the scanning (10) providing scan data for a volume of the patient;
segmenting (12), by an image processor (32) of the medical imaging system (31), an anatomic structure of a patient represented in scan data, the scan data of the anatomic structure comprising a first part of the scan data based on the segmenting (12);
transmitting (14) the scan data in at least the first part and a second part to one or more remote server (38)s, the one or more remote server (38)s being remote from the medical imaging system (31), the transmitting (14) prioritizing the transmitting (14) of the first part before the second part;
receiving (16) a first result from the remote server (38) based on image processing of the first part; and
displaying (17) the first result.

2. The method of claim 1 further comprising:
detecting (11) an anatomy of interest from the scan data;
wherein segmenting (12) comprises segmenting (12) the anatomic structure based on the detecting (11) of the anatomy.

3. The method of claim 1 further comprising:
separately compressing (13) the scan data of the first and second parts, wherein transmitting (14) comprises transmitting (14) the first and second parts as compressed.

4. The method of claim 1 further comprising receiving (16) a second result from the remote server (38) based on image processing of the second part.

5. The method of claim 1 wherein receiving (16) the first result occurs prior to completion of the transmitting (14) of the second part.

6. The method of claim 1 wherein segmenting (12) comprises segmenting (12) with a machine-learned network.

7. The method of claim 1 wherein segmenting (12) the anatomical structure comprises segmenting (12) a first organ or a first portion of the first organ, the first part comprising the scan data representing the first organ or the first portion of the first organ, and wherein the second part comprises the scan data representing a segmented second organ or a second part of the first organ.

8. The method of claim 1 wherein transmitting (14) comprises transmitting (14) the first part with information in addition to the scan data, and wherein receiving (16) comprises receiving (16) the first result based on the image processing of the first part and based on the information.

9. The method of claim 1 wherein transmitting (14) comprises transmitting (14) the first part to a first remote server (38) and the second part to a second remote server (38)s, the first and second remote server (38)s selected based on the anatomic structure in the first part and based on another anatomic structure in the second part.

10. The method of claim 1 further comprising receiving (16) a request from the one or more remote server (38)s for additional information and providing by the medical imaging system (31) the additional information.

11. A method for context-driven decomposition for network transmission in medical imaging, the method comprising:
detecting (11), by an image processor (32), an abnormality represented in image data of a patient;
segmenting (12), by the image processor (32), a first region of interest in the image data based on the detecting (11) of the abnormality;
transmitting (14) the image data for the region of interest as a file separate from the image data for outside the region of interest to a server (38), the server (38) remote from the image processor (32);
receiving (16) a quantity, classification, segmentation, ranking, or combinations thereof from the server (38) for the abnormality based on the image data for the region of interest; and
displaying (17) the quantity, classification, segmentation, ranking, or combinations thereof.

12. The method of claim 11 wherein detecting (11) comprises detecting (11) the abnormality in an organ by a first machine-learned network, and wherein segmenting (12) comprises segmenting (12) the organ by a second machine-learned network.

13. The method of claim 11 wherein segmenting (12) comprises segmenting (12) the region of interest and other regions of interest, and wherein transmitting (14) comprises transmitting (14) the image data for the region of interest as the separate file and transmitting (14) the image data for the other regions of interest in other files sequentially, the separate file for the region of interest being transmitted before the other files.

14. The method of claim 11 wherein transmitting (14) comprises transmitting (14) the file with information in addition to the image data, and wherein receiving (16) comprises receiving (16) the quantity, classification, segmentation, ranking, or combinations thereof based on image processing of the image data of the region of interest and based on the information.

15. The method of claim 11 wherein transmitting (14) to the server (38) comprises selecting the server (38) from a group of server (38)s based on the region of interest.

16. The method of claim 11 further comprising receiving (16) a request from the server (38) for additional information and providing by the image processor (32) the additional information.

17. A system for context-driven decomposition for network transmission in medical imaging, the system comprising:
a medical imaging system (31) configured to scan a patient and divide the scan data into blocks based on anatomy of the patient, the medical imaging system (31) being at a medical facility, the medical imaging system (31) configured to transmit the blocks in an order from anatomical context of the scan data for the blocks; and
a server (38) connected to the medical imaging system (31) through a network and outside the facility, the server (38) configured to receive the blocks of scan data, image process each block separately, and return information from the image process for each block to the medical imaging system (31).

18. The system of claim 17 wherein the medical imaging system (31) is configured to transmit the blocks as separate files and the anatomical context is a detection of an abnormality in anatomy so that the block representing the abnormality is transmitted before blocks for other organs.

19. The system of claim 17 wherein the medical imaging system (31) is configured to divide by segmenting (12) based on the anatomy and configured to transmit the block with the order based on detection of an abnormality in anatomy.

20. The system of claim 17 wherein the server (38) is configured to request additional scan data relative to one of the blocks and wherein the medical imaging system (31) is configured to provide the additional scan data in response to the request.
